# EUROPEAN PATENT APPLICATION

(11) **EP 1 064 936 A1**
(43) Date of publication of application: **03.01.2001**
(21) Application number: 99309717.9
(22) Date of filing: 03.12.1999
(51) Int. Cl.: A61K 9/20, A61K 31/445

(54) **Novel composition of paroxetine methanesulfonate**

(30) Priority: 22.06.1999 GB 9914601; 23.06.1999 GB 9914709; 19.11.1999 GB 9927501
(71) Applicant: SMITHKLINE BEECHAM PLC, Brentford, Middlesex TW8 9EP (GB)
(72) Inventor: Al Deeb Al Ghazawi, Ahmad Khalaf, Smithkline, Harlow, Essex CM19 5AW (GB); Elder, David Philip, SmithKline Beecham Pharmceut., Harlow, Essex CM19 5AW (GB); Meneaud, née Patel, Padma, Smithkline Beecham Phar, Harlow, Essex CM19 5AW (GB)
(74) Representative: Thompson, Clive Beresford

(57) **Abstract**

Pharmaceutical compositions comprising paroxetine methanesulfonate are described.

## Description

The present invention relates to novel pharmaceutical compositions comprising paroxetine methanesulfonate and their use in treating and or preventing medical disorders.

Paroxetine methanesulfonate and pharmaceutical compositions thereof are disclosed in the published PCT application WO 98/56787 (Synthon). The Synthon application discloses pharmaceutical compositions that comprise a carrier or diluent, but the application is silent as to the kind of carrier or diluent which is suitable or that which maximizes the advantages of paroxetine methanesulfonate.

It has now been discovered that paroxetine methanesulfonate can be advantageously formulated with a water-soluble or hydrophilic diluent.

Accordingly, in a first aspect of the present invention there is provided a composition comprising paroxetine methanesulfonate and a pharmaceutically acceptable carrier wherein the carrier comprises a water-soluble and/or hydrophilic diluent, excluding the following tablet compositions consisting of:

| INGREDIENTS | 20 mg Tablet | 30mg Tablet |
|---|---|---|
| Paroxetine Methanesulfonate | 20.00 mg | 30.0 mg |
| | (calc. as free base) | (calc. as free base) |
| Dicalcium Phosphate (DCP) | 83.34 mg | 125.0 mg |
| Microcrystalline Cellulose | 50.67 mg | 76.0 mg |
| Sodium Starch Glycollate | 8.34 mg | 12.5 mg |
| Magnesium Stearate | 1.67 mg | 2.5 mg. |

These tablet compositions consisting essentially of paroxetine methanesulfonate (15% w/w), dibasic calcium phosphate dihydrate (49% w/w), microcrystalline cellulose (30% w/w), sodium starch glycollate (5% w/w) and magnesium stearate (1 % w/w) are thereby disclaimed as such formulations are disclosed in GB-A-2336364.

The use of a water-soluble and/or hydrophilic diluent according to the present invention surprisingly enhances the dissolution rate of paroxetine methanesulfonate.

Suitably the aqueous solubility of a water-soluble diluent at 20°C is at least 0.005 mg/ml, preferably at least 0.01 mg/ml and more preferably at least 0.1mg/ml, for example 0.2 mg/ml. Suitable water-soluble diluents include water-soluble carbohydrate diluents such as sugar or starch diluents and mixtures thereof.

Suitable hydrophilic diluents include carbohydrate hydrophilic diluents such as cellulose diluents.

Carbohydrate diluents suitable for use in the present invention include compressible sugar, confectioner's sugar, dextrates, dextrin, dextrose, fructose, microcrystalline cellulose (such as different grades of Avicel, Emcocel, and Vivacel, e.g. Avicel PH101, Avicel PH 102, Emcocel 50M, Emcocel 90M, Vivacel 101, Vivacel 102), silicified microcrystalline cellulose (which is a mixture of colloidal silicon dioxide and microcrystalline cellulose such as Prosolv SMCC99), pregelatinised starch, powdered cellulose, lactose, maltodextrin, mannitol, sorbitol, sucrose, sugar spheres, lactitol, maltitol or xylitol or mixtures thereof.

Suitably the diluents of the present invention may be present in an amount ranging from 1 to 99%, preferably 10 to 95%, more preferably 20 to 95%, and most preferably from 40 to 95%, for example 80 to 90% w/w of the composition.

If desired the diluent of the present invention may be present in admixture with an additional diluent, such as calcium carbonate, calcium sulfate, dibasic calcium phosphate dihydrate, or dibasic calcium phosphate or a mixture thereof. Suitably such diluent admixture comprises at least 20% by weight (e.g. 30%, 40%, 50%, 60%, 70%, 80%, or 90% by weight) of the water-soluble and/or hydrophilic diluent. Preferably the water-soluble and/or hydrophilic diluent is present as the sole diluent.

Suitably compositions of the present invention may also include a binder, a disintegrant, a lubricant, a glidant, a surfactant, a colouring agent, and a flavouring agent. These agents may be utilized in a conventional manner, for example in a manner similar to that already used for marketed paroxetine formulations. Examples of such excipients are described in the Handbook of Pharmaceutical Excipients (Second Edition, 1994, edited by A. Wade and P. Weller, published by the American Pharmaceutical Association and the Pharmaceutical Press).

Suitably the compositions of the present invention comprise up to 30% by weight of a disintegrant, preferably from 1 to 20%, more preferably from 2-10% and even more preferably from 4-8% by weight of the composition.

Suitable disintegrants may be selected from alginic acid, carboxymethylcellulose calcium, carboxymethylcellulose sodium, colloidal silicon dioxide, croscarmellose sodium, crospovidone, guar gum, magnesium aluminium silicate, microcrystalline cellulose, methyl cellulose, polacrilin potassium, pregelatinised starch, sodium alginate, sodium lauryl sulphate, sodium starch glycollate, starch, carmelose sodium, cationic exchange resins, modified starch, sodium glycine carbonate and mixtures thereof. Preferred disintegrants include starch, methylcellulose, crospovidone, croscarmellose sodium and sodium starch glycollate and mixtures thereof. Most preferably the disintegrant is sodium starch glycollate.

The compositions of this invention may comprise paroxetine methanesulfonate in non-crystalline form, preferably in crystalline form, including any solvates or hydrates thereof.

Paroxetine methanesulfonate exists in more than one crystalline form. For example W098/56787 describes a crystalline form of paroxetine methanesulfonate having *inter alia* one or more of the following characteristic Infra Red (IR) peaks: 1208, 1169, 1038, 962, 931, 838 and 546 cm⁻¹. Another crystalline form of paroxetine methanesulfonate is disclosed in GB-A-2336364 having *inter alia* one or more of the following characteristic IR peaks: 1604, 1194, 1045, 946, 830, 601, 554 and 539 cm⁻¹.

Suitably the compositions are usually presented as unit dose compositions containing from 1 to 200mg of active ingredient calculated on a free base basis, more usually from 5 to 100mg, for example 10 to 50mg such as 10, 12.5, 15, 20, 25, 30 or 40mg per unit dose. Most preferably unit doses contain 20mg of active ingredient calculated on a free base basis. Such a composition is normally taken by a human patient from 1 to 6 times daily, for example 2, 3 or 4 times daily so that the total amount of active agent administered is within the range 5 to 400mg of active ingredient calculated on a free base basis. Thus a suitable daily dose is from 0.05 to 6mg/kg, more preferably 0.14 to 0.86 mg/kg. Most preferably the unit dose is taken once a day.

Preferred unit dosage forms are usually adapted for oral administration, for example tablets or capsules, especially a modified oval or pentagonal shaped tablet.

The compositions of this invention may be formulated by conventional methods of admixture such as blending, filling and compressing.

For example tablets can be produced by a wet granulation process. Suitably the active drug substance and excipients are screened and mixed in a high shear mixer granulator. The blend is granulated by the addition of a granulating solution (typically purified water, disintegration agent dissolved/dispersed in purified water, or drug dissolved/dispersed in purified water or a suitable solvent) sprayed into the high shear mixer granulator. If desired wetting agents e.g. surfactants can be added. The resulting granules are dried usually with residual moisture of 1-5 % by tray, fluid bed or microwave drying techniques. The dried granules are milled to produce a uniform particle size, the granules are blended with extragranular excipients as necessary, typically a lubricant and glidant (e.g. magnesium stearate, silicon dioxide). The compression blend can be compressed using a rotary tablet press typically in the range of 100 to 1000mg. The resulting tablets can be coated in a pan coater typically with a 1-5% aqueous film coat.

Alternatively tablets can be produced by a direct compression process. Suitably the active drug substance and excipients are screened and mixed in a suitable blender e.g. a cone, cube or V- blender. Other excipients are added as necessary, and further blended. The compression blend can be compressed using a rotary tablet press typically in the range of 100 to 1000mg. The resulting tablets can be coated in a pan coater.

Suitably capsules can be produced by screening and mixing the active drug substance and excipients in a suitable blender e.g. a cone, cube or V- blender. Other excipients are added as necessary, typically a lubricant and glidant, and the mixture blended. The blend is filled into capsules with a fill weight typically ranging from 100-1000mg using a standard capsule filling machine.

The compositions may be used to treat and prevent the following disorders:

| | |
|---|---|
| Alcoholism | Anxiety |
| Depression | Obsessive Compulsive Disorder |
| Panic Disorder | Chronic Pain |
| Obesity | Senile Dementia |
| Migraine | Bulimia |
| Anorexia | Social Phobia |
| Pre-Menstrual Dysphoric Disorder (PMDD) | Adolescent Depression |
| Smoking Cessation | |

These disorders are herein after referred to as "the Disorders".

The present invention further provides a method for treating and/or preventing any one or more of the Disorders by administering an effective and/or prophylactic amount of a composition of the invention to a sufferer in need thereof.

The present invention also provides the use of a diluent of the invention in the manufacture of a paroxetine methanesulfonate containing medicament for treating and/or preventing the Disorders.

The following Examples illustrate the present invention.

### Examples 1 to 9

The following nine paroxetine methanesulfonate formulations (each containing 20mg paroxetine free base) were prepared using a direct compression process as hereinbefore described and compressed into tablets each weighing 350 mg. The rates of dissolution of these formulations were compared with a control tablet formulation comprising paroxetine hydrochloride hemihydrate (also containing 20mg paroxetine free base) which salt is used in commercial presentations of paroxetine.

| EXAMPLE (% by weight) | Control | 1 | 2 | 3 | 4 | 5 | 6 | 7 | 8 | 9 |
|---|---|---|---|---|---|---|---|---|---|---|
| Paroxetine HCI Hemihydrate | 6.51 | ------ | ------ | ------ | ------ | ------ | ------ | ------ | ------ | ------ |
| Paroxetine Methanesulfonate # | ------ | 7.38 | 7.38 | 7.38 | 7.38 | 7.38 | 7.38 | 7.38 | 7.38 | 7.38 |
| Dibasic Calcium phosphate | 90.79 | 89.92 | ------ | ------ | ------ | ------ | ------ | 87.62 | ------ | ------ |
| Lactose - Fast Flow | ------ | ------ | 89.92 | ------ | ------ | ------ | ------ | ------ | 87.62 | 87.33 |
| Micro-crystalline cellulose | ------ | ------ | ------ | 89.92 | ------ | ----- | ------ | ------ | ------ | ------ |
| Mannitol SD | ------ | ------ | ------ | ------ | 89.92 | ------ | ------ | ------ | ------ | ------ |
| Silicified micro-crystalline cellulose | ------ | ------ | ------ | ------ | ------ | 89.92 | ------ | ------ | ------ | ------ |
| Lactitol | ------ | ------ | ------ | ------ | ------ | ------ | 89.92 | ------ | ------ | ------ |
| Sodium starch glycollate | 1.70 | 1.70 | 1.70 | 1.70 | 1.70 | 1.70 | 1.70 | 4.00 | 400 | 4.00 |
| Sodium lauryl sulphate | ------ | ------ | ------ | ------ | ------ | ------ | ------ | ------ | ------ | 0.29 |
| Magnesium stearate | 1.00 | 1.00 | 1.00 | 1.00 | 1.00 | 1.00 | 1.00 | 1.00 | 1.00 | 1.00 |
| Total | 100.00 | 100.00 | 100.00 | 100.00 | 100.00 | 100.00 | 100.00 | 100.00 | 100.00 | 100.00 |

| | | | | | | | | | | |
|---|---|---|---|---|---|---|---|---|---|---|
| # Crystalline paroxetine methanesulfonate as described in GB-A-2336364. | | | | | | | | | | |

The dissolution profiles of the test formulations were assessed using USP apparatus II (paddles) rotating at 60 rpm in 0.1 M hydrochloric acid (900 ml). Quantification is determined by UV spectroscopy. The following results were obtained.

| Example | SPEED OF DISSOLUTION (versus control) | DISSOLUTION % drug released after : | | | |
|---|---|---|---|---|---|
| | | 15 min. | 30 min. | 45 min. | 60 min. |
| | | Mean | Mean | Mean | Mean |
| Control | N/A | 73 | 97 | 103 | 104 |
| 1 | S | 27 | 59 | 82 | 98 |
| 2 | F | 87 | 103 | 103 | 104 |
| 3 | F | 107 | 105 | 104 | 104 |
| 4 | F | 106 | 107 | 106 | 106 |
| 5 | F | 99 | 101 | 101 | 102 |
| 6 | F | 97 | 97 | 97 | 97 |
| 7 | S | 70 | 88 | 91 | 93 |
| 8 | F | 99 | 102 | 102 | 102 |
| 9 | F | 98 | 99 | 99 | 99 |

- NA: not applicable
- S: slower than control formulation
- F: faster than control formulation

The formulation of Example 1 is essentially identical to the control formulation except for the replacement of paroxetine hydrochloride hemihydrate with paroxetine methanesulfonate. Whilst the methanesulfonate salt of paroxetine is significantly more soluble than the hydrochloride salt, surprisingly the rate of dissolution of Example 1, which comprises dibasic calcium phosphate as the sole diluent, is much slower than the control formulation. This observation of reduced dissolution from a salt with higher aqueous solubility than the standard hydrochloride hemihydrate salt can be explained by the in *situ* formation of the hydrochloride hemihydrate salt on the dissolving surface of the tablet of Example 1. This hydrochloride hemihydrate salt forms a substantial shield on the tablet surface which then needs to dissolve in turn and the whole dissolution process is slowed.

By contrast the dissolution rates of Examples 2 to 6, which contain a carbohydrate diluent instead of dibasic calcium phosphate, are faster than the control formulation. Whilst the *in situ* formation of the hydrochloride hemihydrate salt will occur the presence of the carbohydrate diluent provides an improved dissolution rate.

The results for Examples 1 and 7 indicate that the rate of dissolution for paroxetine methane sulfonate formulations containing dibasic calcium phosphate can be increased by raising the level of disintegrant present. However it is preferable also to use a carbohydrate diluent as evidenced by the results for Examples 8 and 9 which dissolution rates are also faster than the control formulation.

In summary, these results demonstrate that the highly water soluble paroxetine methanesulfonate salt can be advantageously formulated with a water-soluble and/or hydrophilic diluent (such as a carbohydrate diluent).

## Claims

1. A pharmaceutical composition comprising paroxetine methanesulfonate and a pharmaceutically acceptable carrier wherein the carrier comprises a water-soluble and/or hydrophilic diluent, excluding the following tablet compositions consisting of:
| INGREDIENTS | 20 mg Tablet | 30mg Tablet |
|---|---|---|
| Paroxetine Methanesulfonate | 20.00 mg | 30.0 mg |
| | (calc. as free base) | (calc. as free base) |
| Dicalcium Phosphate (DCP) | 83.34 mg | 125.0 mg |
| Microcrystalline Cellulose | 50.67 mg | 76.0 mg |
| Sodium Starch Glycollate | 8.34 mg | 12.5 mg |
| Magnesium Stearate | 1.67 mg | 2.5 mg. |

2. A composition according to claim 1 wherein the diluent has a water solubility at 20°C of at least 0.005 mg/ml.

3. A composition according to claim 2 wherein the diluent has a water solubility at 20°C of at least 0.01 mg/ml.

4. A composition according to claim 3 wherein the diluent has a water solubility at 20°C of at least 0.1 mg/ml.

5. A composition according to claim 1 wherein the water soluble and/or hydrophilic diluent is a carbohydrate diluent.

6. A composition according to claim 5 wherein the carbohydrate diluent is selected from compressible sugar, confectioner's sugar, a dextrate, dextrin, dextrose, fructose, microcrystalline cellulose, silicified microcrystalline cellulose, pregelatinised starch, powdered cellulose, lactose, maltodextrin, mannitol, sorbitol, sucrose, sugar spheres, lactitol, maltitol, or xylitol or a mixture thereof.

7. A composition according to claim 6 wherein the carbohydrate diluent is selected from lactose, microcrystalline cellulose or mannitol or a mixture thereof.

8. A composition according to any one of claims 1 to 7 wherein the diluent is present in an amount ranging from 1 to 99% w/w of the composition.

9. A composition according to claim 8 wherein the diluent is present in an amount of ranging from 20 to 95% w/w of the composition.

10. A composition according to claim 9 wherein the diluent is present in an amount ranging from 40 to 95% w/w of the composition.

11. A composition according to claim 10 wherein the diluent is present in an amount ranging from 80 to 90% w/w of the composition.

12. A composition according to any one of claims 1 to 11 further comprising an additional diluent selected from calcium carbonate, calcium sulfate, dibasic calcium phosphate dihydrate or dibasic calcium phosphate or a mixture thereof.

13. A composition according to any one of claims 1 to 12 which does not contain dicalcium phosphate in combination with microcrystalline cellulose.

14. A composition according to any one of claims 1 to 12 which does not contain dicalcium phosphate.

15. A composition according to any one of claims 12 to 14 wherein the diluent admixture comprises at least 20 % by weight thereof of the water-soluble and/ or hydrophilic diluent.

16. A composition according to claim 15 wherein the diluent admixture comprises at least 40 % by weight thereof of the water-soluble and/ or hydrophilic diluent.

17. A composition according to claim 15 wherein the diluent admixture comprises at least 60 % by weight thereof of the water-soluble and/ or hydrophilic diluent.

18. A composition according to claim 15 wherein the diluent admixture comprises at least 80 % by weight thereof of the water-soluble and/ or hydrophilic diluent.

19. A composition according to any one of claims 1 to 11 wherein the water soluble and/or hydrophilic diluent is present as the sole diluent.

20. A composition according to any one of claims 1 to 19 further comprising a disintegrant.

21. A composition according to claim 20 wherein the disintegrant is selected from starch, methylcellulose, crospovidone, croscarmellose sodium or sodium starch glycollate or a mixture thereof.

22. A composition according to claim 21 wherein the disintegrant is sodium starch glycollate.

23. A composition according to any one of claims 20 to 22 wherein the disintegrant is present in an amount up to 30% w/w of the composition.

24. A composition according to claim 23 wherein the disintegrant is present from 1 to 20 % w/w of the composition

25. A composition according to claim 24 wherein the disintegrant is present from 2 to 10 % w/w of the composition.

26. A composition according to any one of claims 1 to 25 wherein paroxetine methanesulfonate is in crystalline form.

27. A composition according to claim 26 wherein the paroxetine methanesulfonate has inter *alia* the following characteristic IR peaks: 1208, 1169, 1038, 962, 931, 838 and 546 ± 2 cm⁻¹.

28. A composition according to claim 26 wherein the paroxetine methanesulfonate has *inter alia* one or more of the following characteristic IR peaks: 1604, 1194, 1045, 946, 830, 601, 554 and 539 ± 2 cm⁻¹.

29. A composition according to any one of claims 1 to 28 comprising 1 to 200mg of paroxetine methanesulfonate per unit dose, calculated on a free base basis.

30. A composition according to claim 29 comprising 10 to 50mg of paroxetine methanesulfonate per unit dose, calculated on a free base basis.

31. A composition according to claim 30 comprising 10, 12.5, 15, 20, 25, 30 or 40mg of paroxetine methanesulfonate per unit dose, calculated on a free base basis.

32. A composition according to any one of claims 1 to 31 adapted for oral administration.

33. A composition according to claim 32 which is a tablet or capsule.
